# EUROPEAN PATENT SPECIFICATION

(11) **EP 4 431 132 B1**
(45) Date of publication and mention of the grant of the patent: **20.05.2026**
(21) Application number: 23305335.4
(22) Date of filing: 13.03.2023
(51) Int. Cl.: A61M 5/20, A61M 5/28, A61M 5/315, A61M 5/32

(54) **AN AUTOINJECTOR FOR AUTOMATIC INJECTION OF A PRODUCT INTO AN INJECTION SITE**
AUTOINJEKTOR ZUR AUTOMATISCHEN INJEKTION EINES PRODUKTS IN EINE INJEKTIONSSTELLE
AUTO-INJECTEUR POUR INJECTION AUTOMATIQUE D'UN PRODUIT DANS UN SITE D'INJECTION

(43) Date of publication of application: 18.09.2024
(73) Proprietor: Becton Dickinson France, 38800 Le Pont-de-Claix (FR)
(72) Inventor: FIARD, Michael, 38320 Eybens (FR)
(74) Representative: Germain Maureau

(56) References cited:
- EP-A1- 3 381 490
- US-A1- 2011 202 011
- US-A1- 2018 008 773
- US-A1- 2019 365 999

## Description

The present invention relates to an autoinjector.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

Automatic injection devices are designed for automatic injection of a medical product into an injection site. Autoinjectors usually comprise a housing for receiving a medical container. The medical container has a barrel defining a reservoir for containing the medical product, the barrel having a distal end provided with an injection needle and an opened proximal end receiving a plunger rod for pushing a stopper. The opened proximal end is usually provided with a flange.

Autoinjectors also include a safety shield mechanism moving from an extended to a retracted position to respectively shield or unveil the needle and an injection mechanism for automatically injecting the medical product into an injection site. The injection mechanism usually includes a plunger rod for pushing a stopper inside the barrel of the medical container, and an initially compressed spring for moving the plunger rod in the distal direction. Locking means are provided for maintaining the plunger rod in an initial position in which the plunger rod is axially blocked despite the action of the compressed spring. A release member is typically arranged to release the plunger rod from the locking means and allow the spring to push the plunger rod in the distal direction to perform injection. A predetermined displacement of the safety shield towards the retracted position is required to allow the release member to unlock the locking means and release the plunger rod.

It is known from document US2011202011A1 an autoinjection device having front and rear separable assemblies. The rear assembly contains a drive assembly which is triggered unlatched by rearward movement of an actuating sleeve extending from the front assembly. A locking dial locks the actuating sleeve against rearward movement until the dial is moved to an unlocked position.

To assess the robustness of the autoinjectors, autoinjectors are subjected to drop tests as required in ISO11608. These drop tests usually consist in dropping the autoinjectors at least once from a height of 1 m onto a horizontal floor. There are three drop directions : a drop "cap upward", a drop "cap downward", and a drop with the autoinjector being horizontal. The autoinjectors may be sensitive to the drop test "cap upward".

There is therefore a need for an autoinjector that provides a simple and robust solution for avoiding inadvertent activation of the autoinjector.

The invention relates to an autoinjector, for automatic injection of a product into an injection site, said autoinjector comprising :
a housing extending along a longitudinal axis A and configured to receive a medical container having a barrel defining a reservoir for containing a medical product, said barrel having a distal end provided with an injection needle and an opened proximal end configured to receive a plunger rod for pushing a stopper arranged inside the barrel,
a needle cover coupled to and axially movable with respect to said housing between a first extended position, in which the needle cover at least partially shields the injection needle, a retracted position, in which the needle cover moves proximally with respect to the housing, and a second extended position in which the needle cover moves back in the distal direction to shield the injection needle,
a plunger rod axially movable inside the housing between a storage position and an injection end position distally located relative to the storage position, the plunger rod being configured to push the stopper in order to expel the medical product when moving from the storage position to the injection end position,
a retainer, movable with respect to the plunger rod between a blocking position, in which the retainer blocks the plunger rod in the storage position, and a release position, in which the retainer is moved away from the plunger rod to allow axial displacement of the plunger rod from the storage to the injection end position,
a locker, movable with respect to the retainer between a locking position, in which the locker locks the retainer in the blocking position, and a release position, in which the locker allows movement of the retainer to the release position, movement of the locker from the locking position to the release position being caused by the needle cover moving from the first extended position to the retracted position,
a controller, movable with respect to the housing, between a storage position which the controller prevents activation of the autoinjector and an activated position allowing for activation of the autoinjector, said controller including a grasping member configured for allowing a user to move the controller from the storage position to the activated position,
wherein the locker is connected to the controller such that movement of the controller with respect to the housing between the storage and activated position entails movement of the locker with respect to the housing between a corresponding storage and an activated position, and
the locker including a blocking member configured for abutting against a stop of the housing as long as the locker is in the storage position, such that the locker is prevented from moving to the release position with respect to the retainer, the blocking member of the locker being moved away from said stop of the housing when the locker is moved to the activated position, such that the locker can move to the release position with respect to the retainer.

The autoinjector of the invention thus efficiently prevents inadvertent activation of the injection mechanism. Besides, the retainer is part of the controller instead of being part of the housing; this simplifies the manufacturing process of the housing and makes it easier and cheaper to manufacture. This also involves that the housing does not support the force of the injection spring because said injection spring is supported by the controller / retainer. As a result, the housing may be made of a cheaper material, such as for instance polypropylene.

More specifically, an aspect of the invention is an autoinjector, for automatic injection of a product into an injection site, said autoinjector comprising :
a housing extending along a longitudinal axis A and configured to receive a medical container having a barrel defining a reservoir for containing a medical product, said barrel having a distal end provided with an injection needle and an opened proximal end configured to receive a plunger rod for pushing a stopper arranged inside the barrel,
a needle cover coupled to and axially movable with respect to said housing between a first extended position, in which the needle cover at least partially shields the injection needle, a retracted position, in which the needle cover moves proximally with respect to the housing, and a second extended position in which the needle cover moves back in the distal direction to shield the injection needle,
a plunger rod axially movable inside the housing between a storage position and an injection end position distally located relative to the storage position, the plunger rod being configured to push the stopper in order to expel the medical product when moving from the storage position to the injection end position,
a retainer, radially movable between a blocking position, in which the retainer axially blocks the plunger rod in the storage position, and a release position, in which the retainer is moved away from the plunger rod to allow axial displacement of the plunger rod from the storage to the injection end position,
a locker, axially movable with respect to the retainer between a locking position, in which the locker radially abuts against the retainer to lock the retainer in the blocking position, and a release position, in which the locker allows radial movement of the retainer to the release position, axial movement of the locker from the locking position to the release position being caused by the needle cover moving from the first extended position to the retracted position,
a controller, rotationally movable around the longitudinal axis A with respect to the housing, between a storage position which the controller prevents activation of the autoinjector and an activated position allowing for activation of the autoinjector, said controller including a grasping member configured for allowing a user to rotate the controller from the storage position to the activated position,
wherein the locker is connected to the controller such that rotation of the controller between the storage and activated positions entails rotation of the locker with respect to the housing between corresponding storage and activated positions, and
the locker including a proximal blocking member configured for axially abutting against a distal stop of the housing as long as the locker is in the storage position, such that the locker is prevented from axially moving to the release position with respect to the retainer, the proximal blocking member of the locker being circumferentially moved away from said distal stop of the housing when the locker is rotated to the activated position by the retainer, such that the locker can move axially to the release position with respect to the retainer.

The autoinjector of the invention may further include some or all of the features below.

In an embodiment, the controller includes a distal flange having a distal side and a proximal side, and the housing includes a proximal bearing surface arranged for axially abutting against the distal side of said distal flange, and a distal bearing surface for axially abutting against the proximal side of the distal flange.

The first proximal bearing surface and the distal abutment surface thus guide rotation of the controller around the longitudinal axis A with respect to the housing and axially secure the controller with respect to the housing.

Preferably, the proximal bearing surface is arranged at an axially extending indexation rib, said indexation rib including, axially opposite the proximal bearing surface, a distal end configured for engaging an axial slot of a bottom body of the housing.

Preferably, the proximal bearing surface is arranged at an axially extending blocking rib, said blocking rib including, axially opposite the proximal bearing surface, the distal stop configured for axially abutting against the locker.

Preferably, the proximal bearing surface is arranged at an axially extending mounting rib, said mounting rib including, axially opposite the proximal bearing surface, a distal end configured for axially abutting against a proximal end of a bottom body of the housing.

In an embodiment, the controller includes a proximal flange arranged for closing an opened proximal end of the housing, and the housing includes a proximal bearing surface configured for axially abutting against a distal side of said proximal flange.

In an embodiment, the proximal bearing surface configured for axially abutting against a distal side of said proximal flange is arranged at a first axially extending rib of the housing, and a peripheral edge of the distal flange of the controller includes a cutout for receiving the first axially extending rib through the distal flange.

The cutout is also shaped to allow for rotation of the controller from the storage position to the activated position without being hindered by said first axially extending ribs.

In an embodiment, the controller includes a rotation limiting member having a first lateral side configured for orthoradially abutting against the first axially extending rib of the housing when the controller is in the storage position, and a second lateral side configured for orthoradially abutting against a protrusion of the housing when the controller is in the activated position, and the rotation limiting member connects the distal flange to the proximal flange of the controller.

Thus, the rotation limiting member limits rotation of the controller with respect to the housing between the storage and activated positions, but also avoids torsion of the controller by rigidifying the connection between the distal flange and the proximal flange of the controller. Besides, the first axially extending rib accordingly limits rotation of the controller in a first direction, which may be a counterclockwise direction, while the protrusion limits rotation of the controller in a second opposite direction, which may be a clockwise direction.

Possibly, the rotational blocking member is an axial wall extending in a central longitudinal plane. The axial walls radially protrudes from a lateral wall of an axial sleeve configured for receiving the plunger to a peripheral edge of the distal flange and of the proximal flange. Possibly, the proximal flange and the distal flange radially outwardly protrude form said axial sleeve. Possibly, the axial sleeve includes a proximal end that proximally protrudes from the proximal flange and extends outside the housing.

Possibly, the protrusion of the housing defines the distal bearing surface for axially abutting against the proximal side of the distal flange and, axially opposite said distal bearing surface, the protrusion includes a ramp configured for allowing passage of the distal flange over said protrusion during assembly of the controller within the housing.

In an embodiment, the grasping member of the controller includes a tapered outer end configured to point at a first or second indicator of the housing for indicating to the end user when the controller is in the storage position or activated position.

In an embodiment, the controller includes a resiliently deformable holder configured to abut against the housing in order to hold the controller in the storage position before use of the autoinjector.

In an embodiment, the resiliently deformable holder is configured for abutting against a first side of a first axially extending rib of the housing and, opposite said first side, the first axially extending rib of the housing includes an inclined surface, such as a chamfer, for allowing the resiliently deformable holder to deform so as to pass over the first axially extending rib during assembly of the controller within the housing.

In an embodiment, the retainer includes an axially extending guiding leg configured for engaging an axially extending guiding groove of the locker, the axially guiding leg defining an orthoradial pushing surface configured for abutting against a lateral side of the guiding groove to rotate the locker to the activated position when the retainer is rotated to the activated position.

The locker extends around the retainer. Opposite the orthoradial pushing surface, the guiding leg may include a second orthoradial pushing surface for pushing the locker in the opposite rotational direction. The axially extending guiding leg of the retainer and the guiding groove of the locker may be complementarily shaped. Thus, the guiding leg of the retainer forms a rotation driving member cooperating with the locker to rotate the locker from the storage position to the activated position.

In an embodiment, the retainer includes a resiliently deformable inflexion point arranged at the guiding leg for axially abutting against a proximal end of the locker to provide the end user with tactile indication that the injection is about to be triggered.

In an embodiment, the retainer includes resiliently deformable blocking legs including a radially inward protrusion configured for engaging a distal abutment surface of the plunger rod when the retainer is in the blocking position, an opposite radially outward protrusion configured for engaging an axially extending locking groove of the locker such that the outward protrusion abuts against a lateral abutment surface of said locking groove when the locker is in the locking position, and wherein the outward protrusion further defines a proximal shoulder configured for axially abutting against a distal stop inwardly radially protruding from the locking groove.

The blocking legs of the retainer thus form a blocking member radially movable between a blocking position, in which the blocking member axially abuts against the plunger rod for axially blocking the plunger rod in the storage position, and a release position, in which the blocking member is away from the plunger rod to allow for axial movement of the plunger rod from the storage to the injection end position.

In the locking position, the lateral abutment surface of the locker radially abuts against the blocking member of the retainer to maintain the blocking member in the blocking position. In the release position, the lateral abutment surface of the locker is away from the blocking member of the retainer.

Preferably, the retainer is connected to the controller such that rotation of the controller between the storage and activated positions entails rotation of the retainer with respect to the housing between corresponding storage and activated positions, and wherein the locker is connected to the retainer such that rotation of the retainer between said storage and activated positions entails rotation of the locker with respect to the housing between a storage and an activated position.

In an embodiment, the retainer and the controller are made of a single piece.

The locker, the retainer, and/or the controller may have a same angular rotation between the storage and the activated position. They may rotate altogether, simultaneously.

The invention and the advantages arising therefrom will clearly emerge from the detailed description that is given below with reference to the appended drawings as follows :
- Figure 1 is a perspective view of an autoinjector according to an embodiment of the invention, the controller being in the initial position,
- Figure 2 is an exploded view of an autoinjector according to an embodiment of the invention,
- Figure 3 is a perspective view of an autoinjector according to an embodiment of the invention, the top body and the bottom body being removed,
- Figure 4 is a perspective view of an autoinjector according to an embodiment of the invention, the top body, the bottom body and the cam being removed,
- Figure 5 is a perspective view of a top body of a housing of an autoinjector according to an embodiment of the invention,
- Figure 6 is a cross-section view of a top body of a housing of an autoinjector according to an embodiment of the invention,
- Figure 7 is a perspective view of a controller of an autoinjector according to an embodiment of the invention,
- Figure 8 is a side view of a controller of an autoinjector according to an embodiment of the invention,
- Figure 9 is a perspective view of a distal portion of a controller of an autoinjector according to an embodiment of the invention,
- Figure 10 is a cross-section view of an autoinjector according to an embodiment of the invention,
- Figure 11 is a cross-section view of an autoinjector according to an embodiment of the invention,
- Figure 12 is a cross-section view of an autoinjector according to an embodiment of the invention,
- Figure 13 is a perspective view of a locker of an autoinjector according to an embodiment of the invention,
- Figure 14 is a bottom view of a locker of an autoinjector according to an embodiment of the invention,
- Figure 15 is a top view of a locker of an autoinjector according to an embodiment of the invention,
- Figure 16 is a cross-section view of an autoinjector according to an embodiment of the invention,
- Figure 17 is a cross-section view of the autoinjector of Figure 6, at 90° from the longitudinal plane of Figure 6,
- Figure 18 is a cross-section view, in a transverse plane, of an autoinjector according to an embodiment of the invention,
- Figure 19 is a cross-section view, in a transverse plane, of an autoinjector according to an embodiment of the invention, the controller being in the initial position,
- Figure 20 is a perspective view of an autoinjector according to an embodiment of the invention, the controller being in the activated position,
- Figure 21 is a cross-section view, in a transverse plane, of an autoinjector according to an embodiment of the invention, the controller being in the activated position,
- Figure 22 is a cross-section view of an autoinjector according to an embodiment of the invention, the locker being in the intermediate position,
- Figure 23 is a cross-section view of an autoinjector according to an embodiment of the invention, the locker being in the release position.

The different features of the embodiments can be used in combination with and used with other embodiments as long as the combined parts are not inconsistent with or interfere with the operation of the device and assembly. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure.

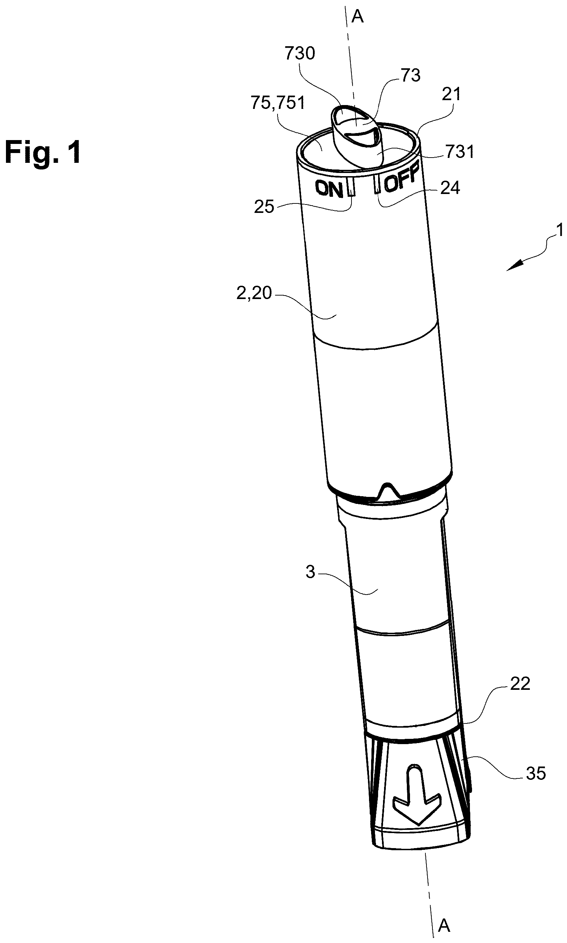

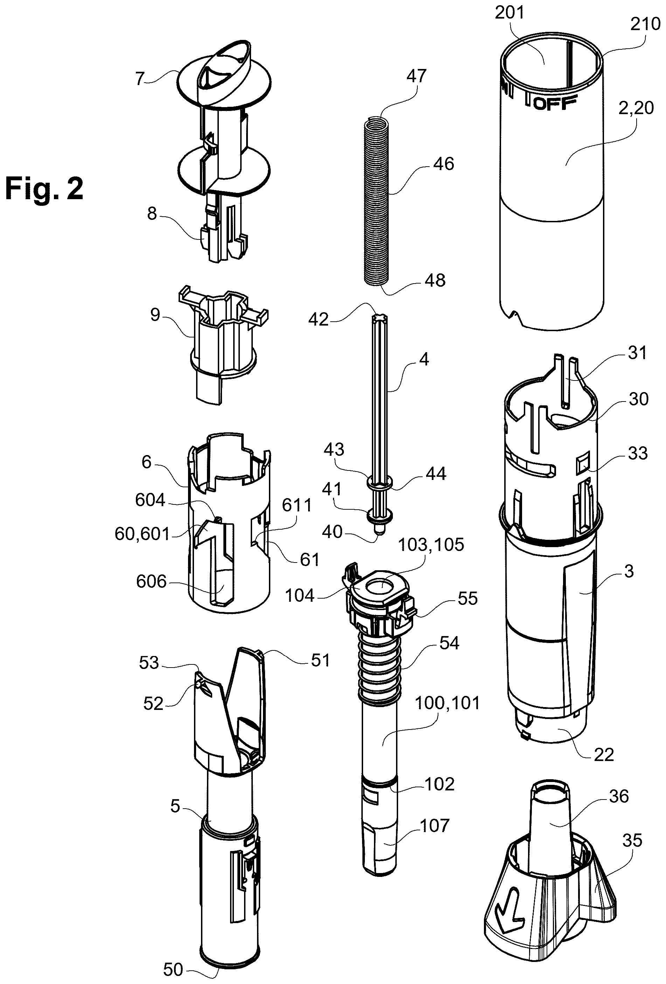

With reference to Figures 1 and 2 is shown an autoinjector 1 according to an embodiment of the invention. The autoinjector 1 is designed for automatic injection of a product into an injection site. The autoinjector 1 extends along a longitudinal axis A. The autoinjector 1 includes a housing 2 comprising a top body 2 and a bottom body 3 assembled to each other by any appropriate securing means such as, for instance, snap-fitting means. The top body 2 and the bottom body 3 are axially and rotationally fixed with one another. The housing 2 has a proximal end 21, which is a proximal end 21 of the top body 2, and an opposite distal end 22, which is a distal end 22 of the bottom body 3. A cap 35 is removably attached to the distal end 22 of the housing 2. The cap 35 may include a remover 36, Figure 2, for removing a needle shield 107 when the cap 35 is withdrawn from the housing 2.

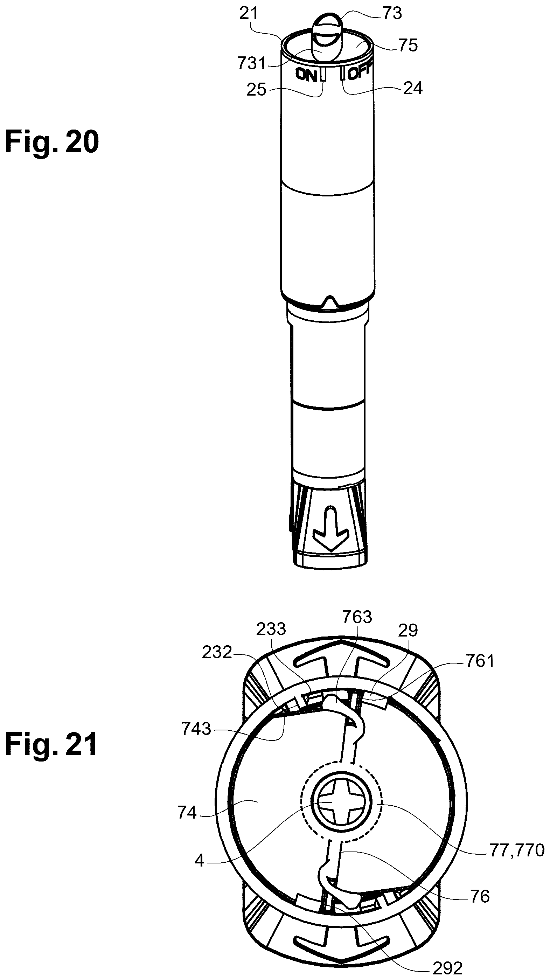

The top body 2 has a cylindrical lateral wall 20 whose proximal end 21 defines a proximal opening 210. The proximal opening 210 may be closed by a top cap which may preferably be formed by a controller 7 as will be explained below. Adjacent to its proximal end 21, and on an outer surface of the lateral wall 20, the top body 2 may include one or more indicators 24, 25, Figure 1, for indicating a position of the controller 7 to the end user. With reference to Figures 1 and 20, the top body 2 of the housing 2 may include a first indicator 24 and/or a second indicator 25 for indicating to the user that the controller 7 is, respectively, in the storage or activated position. The first and second indicators 24, 25 may be arranged at an outer surface of the lateral wall 20 of the housing 2, and preferably adjacent the proximal end 21 of the housing 2 so that the proximal portion of the controller 7 may point at the first indicator 24 or the second indicator 25 depending on the controller's 7 current position. The first and second indicators 24, 25 include any visible mark such as, for instance, an axial recess or rib and/or numbers, colors, or letters such as "OFF", "ON".

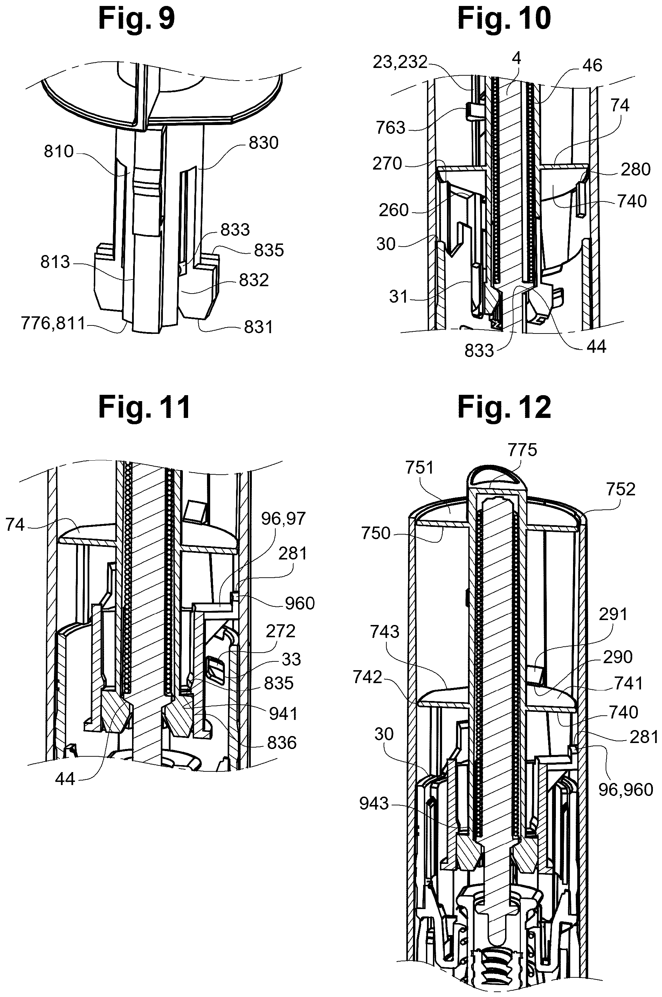

With reference to Figure 2, the bottom body 3 is configured to receive a medical container 100, such as a syringe, preferably a prefilled syringe. The medical container 100 has a tubular barrel 101 defining a reservoir for containing a medical product to be injected. The barrel 101 has a distal end 102 provided with an injection needle (not shown), and a needle shield 107 removably attached to said distal end 102 for protecting and sealing the injection needle. The barrel 101 has an opened proximal end 103 which may be provided with a flange 104. This proximal end 103 defines a proximal opening 105 configured to receive a plunger rod 4 for pushing a stopper 108, Figure 12, arranged inside the barrel 101 in order to expell a medical product contained within the reservoir.

The plunger rod 4 is urged in the distal direction by biasing means, such as an injection spring 46 having a proximal end 47 and an opposite distal end 48. Preferably, the injection spring 46 extends around the plunger rod 4. In another embodiment (not shown), the injection spring 46 may extend inside the plunger rod 4. The distal end 48 of the injection spring 46 axially abuts against an intermediate outward flange 43 of the plunger rod 4 and the proximal end 47 abuts against a transversal wall 775 of the controller 7, Figure 12. A ring 55 may be secured to the proximal end 103 of the barrel 101 and may serve as a support for a safety spring 54 configured for pushing a needle cover 5 in the distal direction.

As visible on Figures 2-3, the autoinjector 1 may also include a rotative cam 6, a locker 9 for axially locking or releasing the plunger rod 4, and the controller 7 which here also forms the top cap.

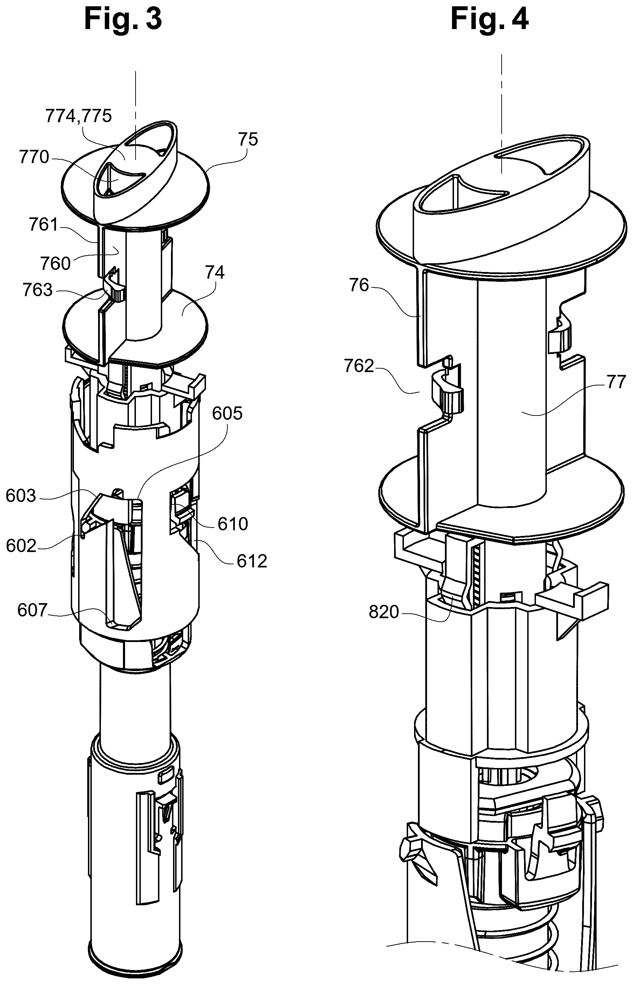

With reference to Figures 2 and 3, the needle cover 5 is in the form of a tubular sleeve configured for accommodating the medical container 100. The needle cover 5 includes a distal end 50 configured for being pressed against an injection site, and an opposite proximal end 51 configured for cooperating with the cam 6 and the locker 9, as shown in Figure 3. The needle cover 5 is axially movable along the longitudinal axis A with respect to the housing 2 between a first extended position (pre-use position, Figure 3), in which the needle cover 5 at least partially or completely shields the injection needle, a retracted position (not shown), proximally located relative to said first extended position, in which the needle cover 5 causes triggering of the injection (by pushing the locker 9 to a release position, as will be described below), and a second extended position (safety position, not shown) in which the needle cover 5 moves back in the distal direction to safely shield the injection needle. Movement of the needle cover 5 from the first extended position to the retracted position is caused by the user pressing the distal end of the needle cover 5 against the injection site, while movement of the needle cover 5 from the retracted position to the safety position is due to the safety spring 54 pushing the needle cover 5 back in the distal direction when the user removes the injection needle from the injection site.

The needle cover 5 includes a radial tab 52, which may be arranged at the proximal end 51, for engaging a first slot 60 of the cam 6. Therefore, axial movement of the needle cover 5 from the first extended position to the retracted position may cause rotation of the cam 6.

The needle cover 5 includes a proximal pushing surface 53, which may be arranged at the proximal end 51, for abutting against a distal abutment surface 982 of the locker 9. Therefore, axial movement of the needle cover 5 from the first extended position to the retracted position may cause axial movement of the locker 9 in the proximal direction. The contact between the needle cover 5 and the locker 9 is preferably direct, i.e. without any intervening parts. The proximal pushing surface 53 may extend in a circumferential direction. The proximal pushing surface 53 is preferably the proximal-most surface of the needle cover 5.

Still with reference to Figures 2-3, the cam 6 may be in the form of a cylindrical ring, which may receive the medical container 100, the proximal end 51 of the needle cover 5 and a distal end 91 of the locker 9. The cam 6 is provided with a first slot 60 and a second slot 61. The first slot 60 has an oblique portion 601 extending between a first end 602 and a second end 603, a transversal portion 604 extending from the second end 603 to a third end 605, and an axial portion 606 distally extending from the third end 605 to a fourth end 607. The first slot 60 is engaged by the radial tab 52 of the needle cover 5, and may be located at the first end 602 when the needle cover 5 is in the first extended position, at the second end 603 and then at the third end 605 when the needle cover 5 is in the retracted position, and close to or at the fourth end 607 when the needle cover 5 is in the safety position. The second slot 61 includes an axial portion 610 including a proximal shoulder 611, and an oblique portion 612 distally extending from said proximal shoulder 611. The second slot 61 is engaged by the ring 55, which extends in the axial portion 610 when the needle cover 5 is in the first extended position and which slides against the oblique portion 612 when the medical container 100 is pushed by the plunger rod 4 to cause penetration of the injection needle into the injection site. The cam 6 is pivotally mounted inside the housing 2. Rotation of the cam 6 is first caused by the radial tab 52 sliding against an upper side of the oblique portion 601 when the needle cover 5 moves from the first extended position to the retracted position, then by the ring 55 sliding against a lower side of the oblique portion 612 when the ring 55 is pushed distally together with the medical container 100 by the injection spring 46. This second rotation of the cam 6 simultaneously causes the radial tab 52 to move from the second end 603 to the third end 605, so that the radial tab 52 now axially faces the axial portion 606 and can slide distally inside the axial portion 606 when the needle cover 5 moves from the retracted position to the safety position, i.e. after injection, when the injection needle is being removed from the injection site.

As visible on Figure 2, the plunger rod 4 has a distal end 40, an opposite proximal end 42 and an intermediate outward flange 43 arranged between the distal end 40 and the proximal end 42.

The distal end 40, which may include a distal flange 41, is configured to push the stopper 108 arranged inside the barrel 101 in order to expel the medical product. The plunger rod 4 is indeed axially movable inside the housing 2 between the initial (storage) position, Figures 10-12, and an injection end position (not shown) distally located relative to the storage position. In the injection end position, the plunger rod 4 may press the stopper 108 against the distal end of the reservoir formed by the barrel 101. Movement of the plunger rod 4 from the storage position to the injection end position is caused by the injection spring 46 pushing said plunger rod 4 in the distal direction.

The intermediate outward flange 43 defines a proximal side, that provides support to the distal end of the injection spring 46, and a distal side, that defines a distal abutment surface 44 configured for abutting against a proximal blocking surface 833 of a retainer 8 for axially blocking the pluner rod in the initial (storage) position of the plunger rod 4. The intermediate outward flange 43 is here arranged near the distal end 40, so that the retainer 8 engages (and is arranged at) a distal portion of the plunger rod 4. In another embodiment (not shown), the intermediate outward flange 43, and thus the distal abutment surface 44, may be located near the proximal end 42 so that the retainer 8 engages (is arranged at) a proximal portion of the plunger rod 4. In other embodiments, instead of an intermediate outward flange 43, the plunger rod 4 may include a circumferential groove that defines the distal abutment surface 44. Preferably, the distal abutment surface 44 has a frustoconical shape so as to be inclined with regards to the longitudinal axis A.

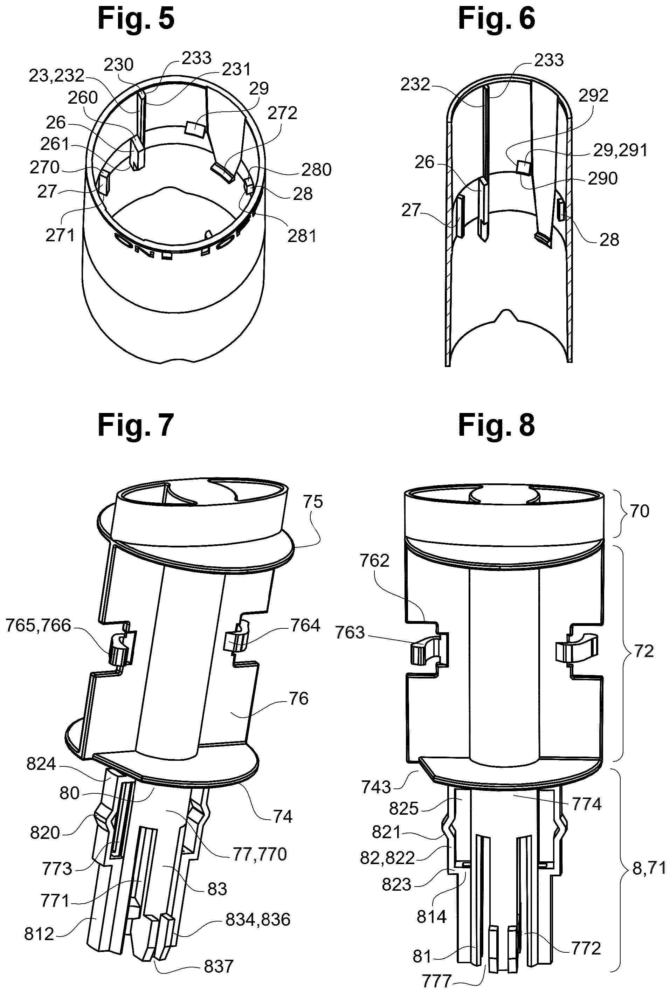

With reference to Figures 7-9, the retainer 8 is in the form of an axial sleeve 77 including a cylindrical lateral wall 770, a proximal end 774 and a distal end 776. The distal end 776 defines a distal opening 777 allowing passage of the plunger rod 4. The lateral wall 770 defines an inner cavity 771 that receives the plunger rod 4 and the injection spring 46. The lateral wall 770 has four axial slots 772 that delimit two diametrically opposite guiding members 81, in the form of guiding legs 81, and two diametrically opposite blocking members 83, in the form of blocking legs 83.

The blocking legs 83 axially extend between a proximal end 830 and a distal end 831, and include an axial blocking surface 833 for axially blocking the plunger rod 4 in the initial position. The blocking legs 83 may further include a lateral blocking surface 836 for radially abutting against the locker 9, and a proximal shoulder 835 for axially abutting against the locker 9. The axial blocking surface 833 may be located at the distal end 831 of the blocking legs 83 and may be defined by an inward protrusion 832 that radially protrudes from an inner surface of the blocking leg 83. The axial blocking surface 833 may be inclined with respect to the longitudinal axis A. The axial blocking surface 833 and the distal abutment surface 44 of the plunger rod 4 may be complementarily shaped. The lateral blocking surface 836 may be defined by an outward protrusion 834 that radially protrude from the blocking leg 83. The outward protrusion 834 is preferably arranged radially opposite the inward protrusion 832. The proximal shoulder 835 is defined by the outward protrusion 834 and may be the proximal-most surface of the outward protrusion 834. The axial blocking leg 83 may actually includes two outward protrusions 834, and thus two lateral blocking surfaces 836 and two proximal shoulders 835, which may be separated by an axial slit 837, as shown on Figures 7-9.

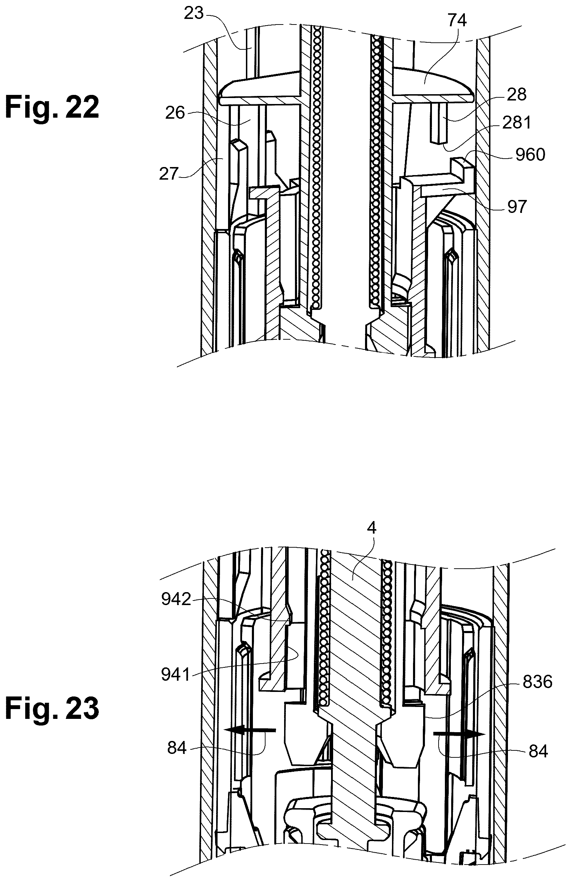

The blocking legs 83 are radially movable, preferably radially deformable, between a blocking position, Figure 10, in which the axial blocking surface 833 axially abuts against the distal abutment surface 44 of the plunger rod 4, so that the plunger rod 4 is blocked in its initial position, and a release position, in which the blocking legs 83 may radially outwardly deform (see the arrows 84 on Figure 23) so that the axial blocking surface 833 is shifted away from the path of the plunger rod 4, thereby allowing the plunger rod 4 to move towards the injection end position. Movement of the blocking legs 83 from the blocking position to the release position is caused by the injection spring 46 acting on the plunger rod 4.

The guiding legs 81 axially extend between a proximal end 810 and a distal end 811, and are configured for guiding axial translation of the locker 9. The guiding legs 81 may include an axial rib 812 protruding from an outer surface thereof. The axial rib 812 is configured for engaging a corresponding axially extending guiding groove 95, Figures 4, 13, 18, of the locker 9, so as to guide axial movement of the locker 9. The axial rib 812 of the retainer 8 may be hollow. The axial rib 812 includes an orthoradial pushing surface 813 configured for abutting against the locker 9, such that rotation of the retainer 8 around the longitudinal axis A causes rotation of the locker 9. That is, the retainer 8 and the locker 9 may rotate together around the longitudinal axis A. As shown on Figures 7-8, the orthoradial pushing surface 813 may delimited by a lateral side of the axial rib 812.

Proximal to the axial rib 812, the guiding legs 81 may include a resilient member configured for opposing movement of the locker 9 in the proximal direction as long as the proximal force exerted on said locker 9 is below a predetermined threshold. This opposition, which is however easily overcome by the end user, helps prevent inadvertent activation of the autoinjector 1 and further provides the end user with a tactile feedback that the injection process is being engaged. The resilient member may be in the form of an axially extending flexible arm 822 including, at an intermediate portion thereof, an outward radial inflexion point 820. The inflexion point 820 defines an inclined distal side 821 configured for abutting against the locker 9, so as to retain the locker 9 in the locking position. The flexible arm 822 has a distal end which may be connected to the proximal end of the axial rib 812 by a first radial arm 823, and a proximal end, which may be connected to the lateral wall 770 of the retainer 8 by a second radial arm 824, Figure 7. The flexible arm 822, the first radial arm 823 and the second radial arm 824 may define a window 825 for allowing inward deformation of the flexible arm 822. The window 825 may open on an axial slit 773 extending through the lateral wall 770 of the retainer 8; the axial slit 773 extends parallel to the flexible arm 822. It is contemplated that the axial length of the retainer 8 in the proximal direction from the inflexion point 820 is sufficient to allow the locker 9 to reach the release position.

The flexible arm 822 is radially movable, more specifically inwardly radially deformable, between an axial blocking position, in which the inflexion point 820 of the flexible arm 822 can axially block proximal movement of the locker 9 towards the release position, and a retracted position in which the inflexion point 820 of the flexible arm 822 does not block proximal movement of the locker 9 any longer. Movement of the flexible arm 822 from the axial blocking position to the retracted position is caused by the end user pressing the distal end 50 of the needle cover 5 against the injection site, which in turn causes the needle cover 5 to exert a proximal force on the locker 9 such that the opposition of the inflexion point 820 is overcome.

The retainer 8 is rotationally movable within the top body 2 between a storage position, in which the locker 9 cannot move to the release position, and an activated position, that allows the locker 9 to move towards the release position, as will be explained hereinafter. The retainer 8, more specifically its proximal end 774, is connected to the controller 7 such that movement of the controller 7 entails movement of the retainer 8. Therefore, rotation of the controller 7 entails rotation of the retainer 8. The retainer 8 is rotationally fixed with respect to the controller 7, so that the retainer 8 rotates together with the controller 7 around the longitudinal axis A. Preferably, the retainer 8 and the controller 7 are made of a single piece. Movement of the retainer 8 from the storage position to the activated position is accordingly caused by the controller 7 moving between the same storage and activated positions.

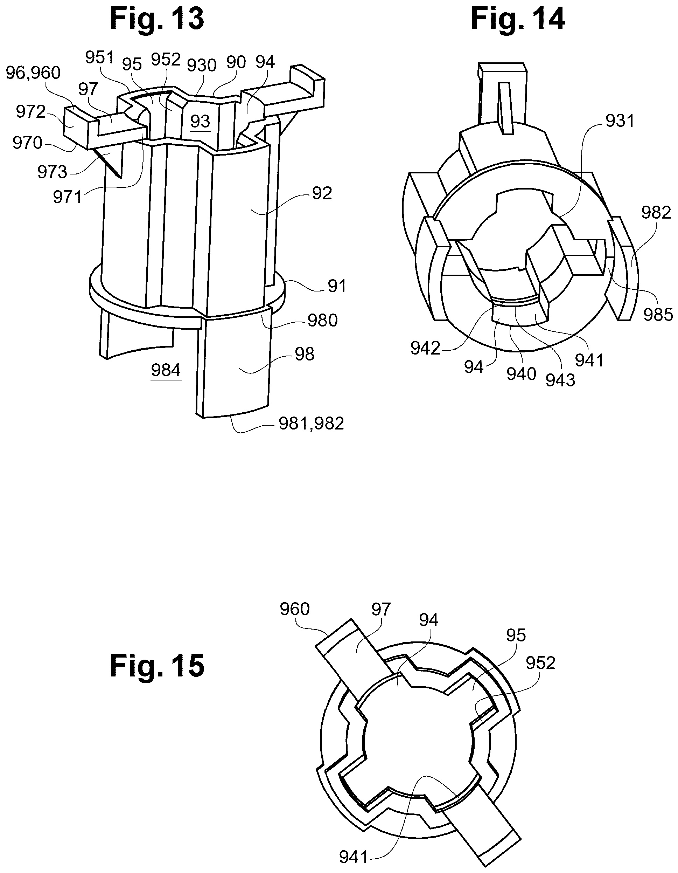

With reference to Figures 13-15, the locker 9 is in the form of a tubular ring having a proximal end 90, a distal end 91 which may be an outer flange, and a lateral wall 92 defining an axial cavity 93 designed to receive the retainer 8, the axial cavity 93 leading to a proximal opening 930 and to an opposite distal opening 931. The axial cavity 93 has two diametrically opposite locking grooves 94 and two diametrically opposite guiding grooves 95. The locking and guiding grooves 94, 95 may be regularly distributed in a circumferential direction, such as 90° from each other.

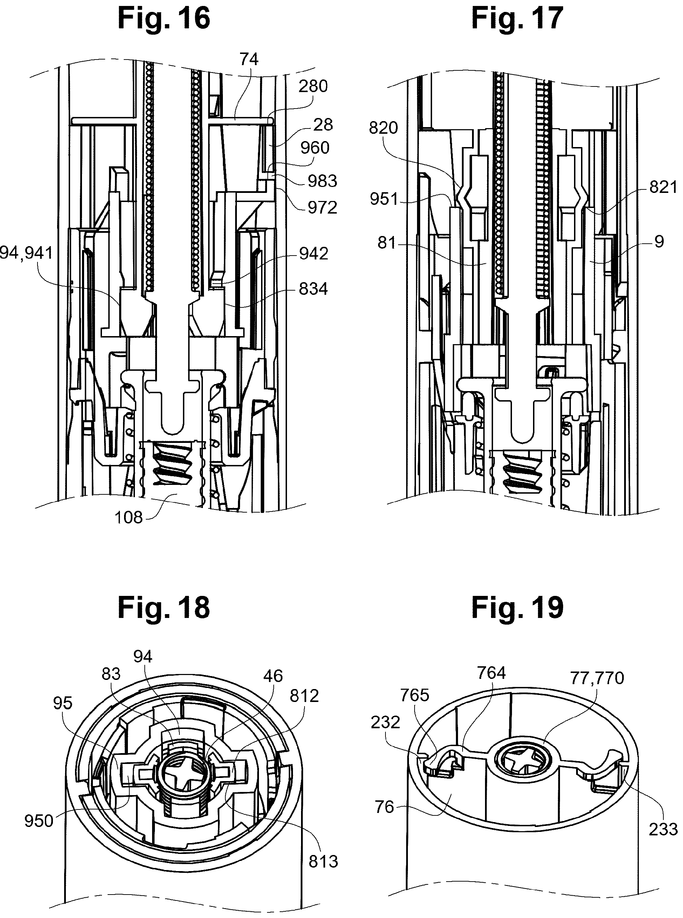

The axially extending locking grooves 94 are configured for receiving the blocking legs 83 of the retainer 8, and more specifically the outward protrusions of said blocking legs 83. As illustrated on Figure 16, the distal end of the locking grooves 94 defines a lateral abutment surface 941 for radially abutting against the lateral blocking surface 836 of the blocking legs 83, such that the blocking legs 83 of the retainer 8 cannot move to the release position. Proximal to the lateral abutment surfaces 941, the locking grooves 94 may include a distal stop 943 configured for axially abutting against the proximal shoulder 835 of the retainer 8. The distal stop 943 may be defined by a radial protrusion 942 inwardly protruding from an inner surface of the locking groove 94. The axial abutment between the distal stop 943 of the locker 9 and the proximal shoulder 835 of the retainer 8 prevents the locker 9 from moving in the distal direction beyond the locking position of the locker 9.

The axially extending guiding grooves 95 are configured for receiving the guiding legs 81 of the retainer 8, that is the axial rib 812 and (when the locker 9 moves towards the release position) the flexible arm 822 of of said guiding legs 81. In the locking position of the locker 9, the inflexion point 820 of the flexible arm 822 extends outside the guiding groove 95 of the locker 9; in the release position of the locker 9, the inflexion point 820 of the flexible arm 822 may extend inside the guiding groove 95 of the locker 9. The axial rib 812 of the retainer 8 and the guiding groove 95 of the locker 9 are designed to define a radial gap 950 that permits entry of the inflexion point 820 of the flexible within the guiding groove 95 when the locker 9 leaves the locking position and proximally moves towards the release position.

The proximal end 90 of the locker 9 and of the guiding grooves 95 define a proximal shoulder 951 axially facing or abutting against a distal side of the inflexion point 820 when the locker 9 is in the locking position. Thus, the locker 9 in the locking position is axially maintained between the inflexion point 820 and the proximal shoulder 835 of the retainer 8.

The guiding grooves 95 further include an orthoradial abutment surface 952 configured for abutting against the orthoradial pushing surface 813 of the guiding legs 81 so that rotation of the retainer 8 causes the guiding legs 81 to push the locker 9 in the circumferential direction. Thus, the locker 9 rotates together with the retainer 8 around the longitudinal axis A.

With reference to Figure 13, the locker 9 includes a blocking member 96 configured for axially abutting against a distal stop 281 of the housing 2 in order to block movement of the locker 9 in the proximal direction. The axial abutment between the blocking member 96 of the locker 9 and the housing 2 enables to maintain the locker 9 in the locking position. Therefore, the autoinjector 1 cannot be activated. The axial abutment between the locker 9 and the housing 2 is preferably direct, i.e. without any intervening part arranged therebetween. The blocking member 96 may include a proximal blocking surface 960 which may be defined by a distal side of a circumferentially extending rib; the proximal blocking 960 surface may be the proximal-most surface of the locker 9. The circumferentially extending rib may be arranged at an outer end 970 of a supporting arm 97 which radially extends from the tubular ring of the locker 9; the supporting arm 97 includes, opposite its outer end 970, an inner end 971 secured to the proximal end 90 of the locker 9, and this inner end 971 is here located adjacent to the proximal end of the locking grooves 94. The radially extending arm 97 may be plate-shaped and may have a distal side and an opposite proximal side. The circumferentially extending rib proximally protrudes from the proximal side of the supporting arm 97. A triangular plate-shaped buttress 973 may extend between the distal side of the supporting arm 97 and the lateral wall 92 of the locker 9 to distribute the axial forces received by the supporting arm 97 to said lateral wall 92. Preferably, the locker 9 includes two diametrically opposite blocking members 96. With reference to Figure 16, it is further contemplated that the circumferentially extending rib may define a lateral abutment wall 972 configured for radially abutting or sliding against an inner surface of the housing 2. Thus, the locker 9 cannot deviate from the central longitudinal axis A; the blocking member 96 of the locker 9 cannot be radially shifted away from the housing 2. The only way to move the blocking member 96 away from axial abutment against the housing 2 is to rotate the locker 9 around the longitudinal axis A, as will be explained below.

With reference to Figures 13 and 17, the locker 9 includes two diametrically opposite driving members 98 configured for retrieving a proximal push of the needle cover 5 when the needle cover 5 moves to the retracted position, so that the locker 9 is pushed by the needle cover 5 in the proximal direction. The driving members 98 may be in the form of two axially extending legs including a proximal end 980 secured to a distal end 91 of the tubular ring and an opposite distal end 981 which defines a distal abutment surface 982 configured for axially abutting against the proximal pushing surface 53 of the needle cover 5 so that the needle cover 5 can push the locker 9 in the proximal direction when the needle cover 5 moves to the retracted position. The distal abutment surface 982 may be the distal-most surface of the locker 9. The axial abutment between the locker 9 and the needle cover 5 is preferably direct, i.e. without any intervening part arranged therebetween. As illustrated on Figure 16, the proximal pushing surface 53 of the needle cover 5 and the distal abutment surface 982 of the axially extending legs 98 may be initially separated by an axial gap 983. The axially extending legs 98 may be circumferentially arranged at 90° from the locking grooves 94 of the locker 9, so that these axially extending legs 98 do not hinder movement of the blocking legs 83 of the retainer 8 when the blocking legs 83 move to the release position. The axially extending legs 98 thus delimit a circumferential gap 984 between them that allows for outward deformation of the blocking legs 83 of the retainer 8. As illustrated on Figure 14, the distal end of the guiding grooves 95 and the proximal end of the axially extending legs 98 may be separated by a distal shoulder 985.

The locker 9 is axially movable with respect to the retainer 8 between a locking position, in which the lateral abutment surfaces 941 of the locker 9 maintain the blocking legs 83 of the retainer 8 in their locking position, and a release position, proximally located with respect to the locking position and in which the lateral abutment surfaces 941 of the locker 9 are shifted away from the blocking legs 83 so that the blocking legs 83 can move to their release position. Axial movement of the locker 9 from the locking position to the release position is caused by the needle cover 5 proximally pushing the locker 9 when the needle cover 5 moves towards the retracted position.

The locker 9 is rotationally movable with respect to the housing 2 around the longitudinal axis A, together with the retainer 8, between a storage position, in which the blocking member of the locker 9 axially faces the distal stop of the housing 2 so that the locker 9 cannot axially move to its release position, and an activated position, in which the blocking member of the locker 9 is offset the distal stop of the housing 2 so that the locker 9 can axially move to its release position. Movement (rotation) of the locker 9 from the storage position to the activated position is caused by the retainer 8 tangentially pushing the locker 9 when the retainer 8 rotates to its activated position.

It is readily understandable from the above that the locker 9 cannot leave the locking position with respect to the retainer 8 as long as the locker 9 is in the storage position with respect to the housing 2, and that the locker 9 can move from the locking position to the release position with respect to the retainer 8 only after having been rotated to the activated position with respect to the housing 2.

With reference to Figures 7-9, the controller 7 is configured for allowing the end user to rotate the retainer 8 and the locker 9 with respect to the housing 2 from the storage to the activated position. The controller 7 includes a proximal portion 70 which may form a top cap and which is configured for interaction with the end user, a distal portion 71 corresponding to the retainer 8, and a median portion 72 extending between the proximal portion 70 and the distal portion 71, the median portion 72 being configured for connecting the controller 7 to the housing 2 such that the controller 7 is axially fixed with respect to the housing 2 and rotationally movable with respect to the housing 2 around the longitudinal axis A. The median portion 72 includes a proximal flange 75, that separates the proximal portion 70 and the median portion 72, and a distal flange 74, that separates the median portion 72 from the retainer 8. The controller 7 defines an axial sleeve 77 which is configured for accommodating the plunger rod 4 and the injection spring 46, and which may extend throughout the controller 7 from its proximal portion to the retainer 8.

As visible on Figure 1 or 20, the proximal portion 70 is configured to extend outside the housing 2 so that the end user can grasp the controller 7. The proximal portion 70 includes a grasping member 73 which may have an oval shape and which may be in the form of a protrusion that proximally protrudes from a proximal side of the proximal flange 75, said protrusion being designed so that the user can exert a torque on the controller 7 around the longitudinal axis A. The protrusion may include one or more recesses 730 opened in the proximal direction. A proximal end 774 of the axial sleeve 77 may protrude from the proximal flange 75 and may extend into said one or more recesses 730. This proximal end has a lateral wall 770 closed by a transversal wall 775 that defines a support for the proximal end 47 of the injection spring 46, Figure 12. It is contemplated that the proximal portion 70 may have a tapered outer end 731 configured for indicating the position of the controller 7 to the end user. For instance, the tapered outer end 731 may point at a first indicator 24 for indicating that the controller 7 is in the storage position, and/or may point at a second indicator 25 for indicating that the controller 7 is in the activated position.

The proximal flange 75 may be shaped to close the proximal opening 210 of the housing 2. A distal side 750 of the proximal flange 75 may be configured to axially abut against a first proximal bearing 230 surface of the housing 2, Figures 5-6. The proximal side 751 and more specifically the distal side 750 of the proximal flange 75 may be orthogonal to the longitudinal axis A to help guide rotation of the controller 7 with respect to the housing 2. A peripheral edge 752 of the proximal flange 75 may be configured to center and guide rotation of the controller 7 with respect to the housing 2. To that end, the outer diameter of the proximal flange 75 may be slightly lower than the inner diameter of the housing 2.

The median portion 72 extends inside the housing 2 and includes two diametrically opposite axial walls 76, which may radially outwardly protrude from a lateral wall 770 of the cylindrical inner sleeve 77. The axial walls 76 are configured for limiting rotation of the controller 7 inside the housing 2, and thus form rotation limiting members 76. The axial walls 76 may extend between a distal side 750 of the proximal flange 75 to a proximal side 741 of the distal flange 74 of the controller 7 for rigidifying the controller 7 and limiting the torsional deformation of the controller 7 due to the torque exerted by the user.

With reference to Figures 7-8, the axial walls 76 may include a cutout 762 and a resiliently deformable holder 763 extending in said cutout 762. The resiliently deformable holder 763 is configured for opposing rotation of the controller 7 from towards an activated position when the controller 7 is in the storage position. The resiliently deformable holder 763 thus maintains the controller 7 in the storage position as long as the torque exerted on the controller 7 is below a predetermined threshold, thereby preventing inadvertent rotation of the controller 7 to the activated position. When the user rotates the controller 7, the resiliently deformable holder 763 however deforms to allow for rotation of the controller 7 to the activated position. As visible on Figure 19, the resiliently deformable holder 763 may be in the form of a curved blade including an inner end 764 secured to the axial wall 76 and a free outer end 765 which defines an abutment surface 766 for orthoradially abutting against a first orthoradial stop 232 of the housing 2. The first orthoradial stop 232 may be formed by a lateral side of the first axially extending rib 23. The holder 763 is radially deformable between an extended position, Figure 19, in which the holder 763 can abut against the housing 2 to oppose rotation of the controller 7 towards the activated position, and a retracted position, in which the holder 763 deforms radially inwardly to pass beyond the first orthoradial stop 232 of the housing 2 such that the controller 7 can move to the activated position, Figure 21.

The axial walls 76 include a first lateral side 760, configured for abutting against the housing 2 when the controller 7 is in the storage position so that rotation of the controller 7 is blocked in a first direction (Figures 10 and 19), and an opposite second lateral side 761 configured for abutting againt the housing 2 when the controller 7 is in the activated position so that rotation of the controller 7 is blocked in a second direction (Figure 21). Thus, the axial walls 76 limit the rotation of the controller 7 with respect to the housing 2, between said storage and activated positions.

The distal flange 74 includes a proximal side 741 and an opposite distal side 740 which are orthogonal to the longitudinal axis A to guide rotation of the controller 7 with respect to the housing 2. The proximal side 741 may be configured for axially abutting against a distal bearing surface 290 of the housing 2, Figure 11, while the distal side 740 may be configured for axially abutting against a proximal bearing surface 230 of the housing 2, Figure 10. The axial distance between the distal bearing surface 290 and the proximal bearing surface 230 of the housing 2 may be slightly greater than the width of the distal flange 74; thus, the distal bearing surface 290, the proximal bearing surface 230 and the distal flange 74 are configured such that the controller 7 is axially secured to the housing 2, but can rotate around the longitudinal axis A with respect to the housing 2 between the storage and the activated positions. A peripheral edge 742 of the distal flange 74 may be configured to center and guide rotation of the controller 7 with respect to the housing 2. To that end, the outer diameter of the distal flange 74 may be slightly lower than the inner diameter of the housing 2. The distal flange 74 may include a cutout 743 arranged along the peripheral edge 742 for allowing the controller 7 to rotate from the storage to the activated position without being hindered by the housing 2. The cutout 743 is shaped so that the distal flange 74 does not abut against the first orthoradial stop 232 of the housing 2 when the controller 7 is rotated towards the activated position, Figure 10. Besides, the cutout 743 is configured to receive a first axially extending rib 23 of the housing 2 when the controller 7 is assembled to the housing 2; that is, the cutout 743 enables to insert the distal flange 74 within the housing 2 without being blocked by the first axially extending rib 23.

The retainer 8 is rotationally fixed with respect to the distal flange 74 of the controller 7. That is, the retainer 8 rotates together with the controller 7. Preferably, the retainer 8 is the distal portion 71 of the controller 7; the controller 7 and the retainer 8 may be made of a single piece.

As stated above, the controller 7 is rotationally movable with respect to the housing 2 between a storage position, in which the first lateral side of the controller 7 abuts against the housing 2 and the second lateral side is away from the housing 2, and an activated position, in which the first lateral side is away from the housing 2 and the second lateral side of the controller 7 abuts against the housing 2. Movement (rotation) of the controller 7 from the storage position to the activated position is caused by the end user turning the controller 7 by means of the grasping member 73. As a result, the end user who wants to use the autoinjector 1 rotates the controller 7, which consequently causes rotation of the locker 9 from the storage to the activated position by means of the retainer 8.

With reference to Figures 5-6, the housing 2 (top body) is in the form of cylindrical tube including a lateral wall 20, an opened proximal end 21 configured to be closed by the controller 7, and an opened distal end 22 configured to be fixedly or removably secured to the bottom body 3 by snap-fitting means. The top body 2 defines an inner axial cavity 201 for receiving the controller 7, the locker 9, the plunger rod 4, the injection spring 46 and possibly the cam 6.

The housing 2 includes a first proximal bearing surface 230 for supporting the proximal flange 75 of the controller 7. The first proximal bearing surface 230 may be defined at a proximal end of a first axially extending rib 23 of the housing 2. The two sides of this first axial rib 23 also define the first and the second orthoradial stops 232, 233 of the housing 2 for abutting, respectively, against the holder 763 and the axial wall 76 of the controller 7, Figure 19, when the controller 7 is in the storage position. The second lateral side may include a chamfer 231 for easing assembly of the controller 7 inside the top body 2 as will be explained below. Preferably, the top body 2 includes two diametrically opposite first axially extending ribs 23.

The housing 2 includes a second proximal bearing surface 260 for supporting the distal flange 74 of the controller 7. The second proximal bearing surface 260 may be defined at a proximal end of a second axially extending rib 26 of the housing 2. The second axial rib 26, or indexation rib 26, may extend alongside the first axial rib 23, as illustrated on Figure 6. The second proximal bearing surface 260 may be specifically shaped to abut against the portion of the distal flange 74 where the cutout 743 extends. To that end, the radial dimension of the second proximal bearing surface 260 may be greater than the radial dimension of the first or any other proximal bearing surface 230 configured for supporting the controller 7. A distal end 261 of the second axial rib 26 may form an indexing member configured for engaging an opened axial slot 31 at the proximal end of 30 the bottom body 3 in order to make the bottom body 3 get into a predetermined rotational position with respect to the top body 2. Preferably, the top body 2 includes two diametrically opposite second axial ribs 26.

The housing 2 includes a third proximal bearing surface 280 for supporting the distal flange 74 of the controller 7. The third proximal bearing surface 280 may be defined at a proximal end of a third axially extending rib, or blocking rib 28, of the housing 2. A distal end of the third axial rib 28 may define the distal stop 281 axially facing or abutting against the locker 9 when the locker 9 is in the storage position, so that the locker 9 cannot move to the release position. Preferably, the top body 2 includes two diametrically opposite third axial ribs 28.

The housing 2 includes a fourth proximal bearing surface 270 for supporting the distal flange 74 of the controller 7. The fourth proximal bearing surface 270 may be defined at a proximal end of a fourth axially extending rib, or mounting rib 27, of the housing 2. A distal end 271 of the fourth axial rib 27 may define a distal abutment axially abutting against the proximal end 30 of the bottom body 3 when the bottom body 3 is assembled to the top body 2. Preferably, the top body 2 includes two diametrically opposite fourth axial ribs 27. Further, the housing 2 may include one, or two diametrically opposite, clipping elements 272 configured for engaging a lateral opening 33 of the bottom body 3 so that the bottom body 3 is snap-fitted on the top body 2, Figure 11.

It results from the above that the housing 2 includes at least one proximal bearing surface 230, 260, 270, 280 arranged for axially abutting against the controller 7. It is contemplated that the second, third, and fourth proximal bearing surfaces 260, 270, 280 which axially abut against the distal flange 74 of the controller 7 are arranged at a same axial position, i.e. extend in the same transversal plane orthogonal to the longitudinal axis A.

The housing 2 may further include a distal bearing surface 290 arranged for axially abutting against the controller 7, and more specifically here against the distal flange 74 of the controller 7, Figure 11. The distal bearing surface 290 may be defined by a radial protrusion 29, which may further include a proximal ramp 291, arranged axially opposite the distal bearing surface 290, for allowing snap-fitting of the distal flange 74 with respect to the housing 2. That is, the ramp 291 eases insertion of the controller 7 in the top body 2. The protrusion 29 may also define a third orthoradial stop 292 which is configured for abutting against the axial walls 76 of the controller 7 for stopping rotation of the controller 7 in the activated position, Figure 21.

The controller 7 is assembled to the top body 2 as follows. The controller 7 is first inserted within the top body 2, through the opened proximal end 21 of the top body 2, by axial displacement of the controller 7 with respect to the top body 2. To that end, the cutout 743 of the distal flange 74 is axially positioned in front of the first axial rib 23; thus, the cutout 743 allows for insertion of the distal flange 74 in the top body 2. Besides, the controller 7 is rotationally positioned so that the holder 763 faces the second orthoradial stop 233 of the first axial rib 23 (see for instance Figure 21), instead of facing the first orthoradial stop 232. In order to be inserted, the controller 7 is thus get into an intermediate position between the storage and the activated position. The controller 7 can then be axially inserted into the housing 2. This causes abutment of the distal flange 74 against the ramp 291 of the protrusion 29; due to the shape of the ramp 291, the distal flange 74 can deform to distally pass beyond the protrusion 29. The distal flange 74 then comes into axial abutment against the second, third and fourth bearing surfaces 260, 270, 280 of the housing 2; the controller 7 cannot move back in the proximal direction since the distal flange 74 has passed the protrusion 29 and is also accordingly blocked by the distal bearing surface 290 of formed by this protrusion. Meanwhile, the proximal flange 75 has come in axial abutment against the first proximal bearing 230 of the housing 2, and now closes the opened proximal end 21 of the top body 2. Yet, the controller 7 is still in an intermediate rotational position and must now be placed into the storage position. The controller 7 is thus rotated towards the storage position. The holder 763 comes into abutment against the second orthoradial stop 233 formed by at one side of the first axial rib 23. Due to the chamfer 231, the holder 763 can resiliently flex towards its retracted position, so as to pass over the first axial rib 23. The controller 7 can accordingly reach the storage position. The holder 763 (its free end) now extends on the opposite side of the first axial rib 23 and can abut against the first orthoradial stop 232 to maintain the controller 7 in the storage position before the controller 7 is rotated to the activated position by the end user, Figure 19.

The operation of the autoinjector 1 is described below.

Initially (Figures 1, 11-12, 16-17, 19), the controller 7, its retainer 8, and the locker 9 are in the storage position. The tapered outer end 731 of the controller 7 points at the first indicator 24 "OFF" of the housing 2, Figure 1, and the locker 9 cannot move axially with respect to the retainer 8 because of the distal stop 281 of the housing 2, Figure 16. The locker 9 is accordingly blocked in the locking position: the blocking legs 83 cannot disengage the plunger rod 4 which is blocked in the storage position too. The injection cannot be triggered.

In order to perform injection, the user has to rotate the controller 7 from the storage to the activated position. By doing so, the holder 763 inwardly deforms to pass beyond the first axial rib 23 of the top body 2, Figure 19. The user carries on rotating the controller 7 until the axial walls 76 of the controller 7 abut against the protrusion 29 of the top body 2. The controller 7 has reached the activated position. The proximal portion 70 of the controller 7 now points at the second indicator 25 "ON" of the housing 2, Figure 20. Since the retainer 8 is rotationally fixed to the controller 7, the retainer 8 has also rotated from the storage to the activated position. Due to the engagement of the guiding legs 81 of the retainer 8 in the guiding grooves 95 of the locker 9, the locker 9 is caused to rotate together with the retainer 8 from the storage to the activated position. As a consequence, the blocking member 96 of the locker 9 has been moved away from the distal stop 281 of the housing 2. The locker 9 is now free to move in the proximal direction towards the release position.

In order to perform injection, the user also has to remove the cap from the bottom body 3 to unveil the distal end 50 of the needle cover 5. The user can then press this distal end 50 against the injection site. As a result, the needle cover 5 moves in the proximal direction from the first extended position towards the retracted position. By doing so, the proximal pushing surface 53 of the needle cover 5 abuts against the driving legs 98 of the locker 9, so that the needle cover 5 pushes the locker 9 in the proximal direction too, until the locker 9 reaches the relase position. It may be noted that the user has to overcome the resistance of the inflexion point 820 to make the locker 9 leave its locking position. This gives to the end user a tactile indication that injection is about to be triggered.

At this stage, i.e. when the locker 9 has reached the release position, Figure 23, the injection needle has already penetrated into the injection site, and the blocking legs 83 of the retainer 8 are no longer prevented from moving towards their release position. Due to the action of the injection spring 46, the blocking legs 83 outwardly deform and the plunger rod 4 is pushed in the distal direction. The plunger rod 4 thus engages the stopper 108 in the barrel 101 and the medical product is expelled into the injection site.

It is readily understandable from the above description that the autoinjector 1 of the invention permits to provide a simple and robust solution for avoiding inadvertent activation of the autoinjector 1. It is to be understood that the present invention is not limited to the embodiments described above and illustrated in the drawings; rather, the skilled person will recognize that many changes and modifications may be made within the scope of the appended claims.

## Claims

1. Autoinjector (1), for automatic injection of a product into an injection site, said autoinjector (1) comprising :
a housing (2) extending along a longitudinal axis A and configured to receive a medical container (100) having a barrel (101) defining a reservoir for containing a medical product, said barrel (101) having a distal end (102) provided with an injection needle and an opened proximal end (103) configured to receive a plunger rod (4) for pushing a stopper (108) arranged inside the barrel (101), the housing (2) including a stop (281),
a needle cover (5) coupled to and axially movable with respect to said housing (2) between a first extended position, in which the needle cover (5) at least partially shields the injection needle, a retracted position, in which the needle cover (5) moves proximally with respect to the housing (2), and a second extended position in which the needle cover (5) moves back in the distal direction to shield the injection needle,
a plunger rod (4) axially movable inside the housing (2) between a storage position and an injection end position distally located relative to the storage position, the plunger rod (4) being configured to push the stopper (108) in order to expel the medical product when moving from the storage position to the injection end position,
a retainer (8), movable with respect to the plunger rod (4) between a blocking position, in which the retainer (8) blocks the plunger rod (4) in the storage position, and a release position, in which the retainer (8) is moved away from the plunger rod (4) to allow axial displacement of the plunger rod (4) from the storage to the injection end position,
a locker (9), movable with respect to the retainer (8) between a locking position, in which the locker (9) locks the retainer (8) in the blocking position, and a release position, in which the locker (9) allows movement of the retainer (8) to the release position, movement of the locker (9) from the locking position to the release position being caused by the needle cover (5) moving from the first extended position to the retracted position,
a controller (7), movable with respect to the housing (2), between a storage position in which the controller (7) prevents activation of the autoinjector (1) and an activated position allowing for activation of the autoinjector (1), said controller (7) including a grasping member (73) configured for allowing a user to move the controller (7) from the storage position to the activated position, wherein the locker (9) is connected to the controller (7) such that movement of the controller (7) with respect to the housing (2) between the storage and activated position entails movement of the locker (9) with respect to the housing (2) between a corresponding storage and an activated position, and
the locker (9) including a blocking member (96) configured for abutting against the stop (281) of the housing (2) as long as the locker (9) is in the storage position, such that the locker (9) is prevented from moving to the release position with respect to the retainer (8), the blocking member (96) of the locker (9) being moved away from said stop (281) of the housing (2) when the locker (9) is moved to the activated position, such that the locker (9) can move to the release position with respect to the retainer (8).

2. Autoinjector (1) according to the preceding claim, wherein the controller (7) includes a distal flange (74) having a distal side (740) and a proximal side (741), and the housing (2) includes a proximal bearing surface (260, 270, 280) arranged for axially abutting against the distal side (740) of said distal flange (74), and a distal bearing surface (290) for axially abutting against the proximal side (741) of the distal flange (74).

3. Autoinjector (1) according to the preceding claim, wherein the proximal bearing surface (260) is arranged at an axially extending indexation rib (26), said indexation rib (26) including, axially opposite the proximal bearing surface (260), a distal end (261) configured for engaging an axial slot (31) of a bottom body (3) of the housing (2).

4. Autoinjector (1) according to the preceding claim 2, wherein the proximal bearing surface (280) is arranged at an axially extending blocking rib (28), said blocking rib (28) including, axially opposite the proximal bearing surface (280), the distal stop (281) configured for axially abutting against the locker (9).

5. Autoinjector (1) according to the preceding claim 2, wherein the proximal bearing surface (270) is arranged at an axially extending mounting rib (27), said mounting rib (27) including, axially opposite the proximal bearing surface (270), a distal end (271) configured for axially abutting against a proximal end (30) of a bottom body (3) of the housing (2).

6. Autoinjector (1) according to any of the preceding claims, wherein the controller (7) includes a proximal flange (75) arranged for closing an opened proximal end (21) of the housing (2), and the housing (2) includes a proximal bearing surface (230) configured for axially abutting against a distal side (751) of said proximal flange (75).

7. Autoinjector (1) according to the preceding claims 2 and 6, wherein the proximal bearing surface (230) configured for axially abutting against a distal side (751) of said proximal flange (75) is arranged at a first axially extending rib (23) of the housing (2), and a peripheral edge (742) of the distal flange (74) of the controller (7) includes a cutout (743) for receiving the first axially extending rib (23) through the distal flange (74).

8. Autoinjector (1) according to the preceding claim, wherein the controller (7) includes a rotation limiting member (76) having a first lateral side (760) configured for orthoradially abutting against the first axially extending rib (23) of the housing (2) when the controller (7) is in the storage position, and a second lateral side (761) configured for orthoradially abutting against a protrusion (29) of the housing (2) when the controller (7) is in the activated position, and the rotation limiting member (76) connects the distal flange (74) to the proximal flange (75) of the controller (7).

9. Autoinjector (1) according to any of the preceding claims, wherein the grasping member (73) of the controller (7) includes a tapered outer end (731) configured to point at a first or second indicator (24, 25) of the housing (2) for indicating to the end user when the controller (7) is in the storage position or activated position.

10. Autoinjector (1) according to any of the preceding claims, wherein the controller (7) includes a resiliently deformable holder (763) configured to abut against the housing (2) in order to hold the controller (7) in the storage position before use of the autoinjector (1).

11. Autoinjector (1) according to the preceding claim, wherein the resiliently deformable holder (763) is configured for abutting against a first side of a first axially extending rib (23) of the housing (2) and, opposite said first side, the first axially extending rib (23) of the housing (2) includes an inclined surface (231), such as a chamfer, for allowing the resiliently deformable holder (763) to deform so as to pass over the first axially extending rib (23) during assembly of the controller (7) within the housing (2).

12. Autoinjector (1) according to any of the preceding claims, wherein the retainer (8) includes an axially extending guiding leg (81) configured for engaging an axially extending guiding groove (95) of the locker (9), the axially guiding leg (81) defining an orthoradial pushing surface (813) configured for abutting against a lateral side of the guiding groove (95) to rotate the locker (9) to the activated position when the retainer (8) is rotated to the activated position.

13. Autoinjector (1) according to the preceding claim, wherein the retainer (8) includes a resiliently deformable inflexion point (820) arranged at the guiding leg (81) for axially abutting against a proximal end (90) of the locker (9) to provide the end user with tactile indication that the injection is about to be triggered.

14. Autoinjector (1) according to any of the preceding claims, wherein the retainer (8) includes resiliently deformable blocking legs (83) including a radially inward protrusion (832) configured for engaging a distal abutment surface (44) of the plunger rod (4) when the retainer (8) is in the blocking position, an opposite radially outward protrusion (834) configured for engaging an axially extending locking groove (94) of the locker (9) such that the outward protrusion (834) abuts against a lateral abutment surface (941) of said locking groove (94) when the locker (9) is in the locking position, and wherein the outward protrusion (834) further defines a proximal shoulder (835) configured for axially abutting against a distal stop (943) inwardly radially protruding from the locking groove (94).

15. Autoinjector (1) according to any of the preceding claims, wherein the retainer (8) and the controller (7) are made of a single piece.

## Patentansprüche

1. Autoinjektor (1) zum automatischen Injizieren eines Produkts an einer Injektionsstelle, wobei die Vorrichtung (1) Folgendes umfasst:
ein sich entlang einer Längsachse A erstreckendes Gehäuse (2), das so eingerichtet ist, dass es einen Medizinbehälter (100) aufnimmt, der einen Zylinder (101) aufweist, der ein Reservoir zum Enthalten eines Medizinprodukts definiert, wobei der Zylinder (101) ein distales Ende (102) aufweist, das mit einer Injektionsnadel versehen ist, und ein geöffnetes proximales Ende (103), das so eingerichtet ist, dass es eine Kolbenstange (4) zum Drücken eines in den Zylinder (101) angeordneten Stoppers (108) aufnimmt, das Gehäuse (2) enthaltend einen Anschlag (281),
eine in Bezug auf das Gehäuse (2) gekoppelte und axial bewegliche Nadelabdeckung (5) zwischen einer ersten ausgefahrenen Position, in der die Nadelabdeckung (5) die Injektionsnadel mindestens teilweise abschirmt, einer zurückgezogenen Position, in der sich die Nadelabdeckung (5) proximal in Bezug auf das Gehäuse (2) bewegt, und einer zweiten ausgefahrenen Position, in der sich die Nadelabdeckung (5) in distaler Richtung zurückbewegt, um die Injektionsnadel abzuschirmen,
eine axial bewegliche Kolbenstange (4) innerhalb des Gehäuses (2) zwischen einer Aufbewahrungsposition und einer Injektionsendposition, die sich distal zur Aufbewahrungsposition befindet, wobei die Kolbenstange (4) so eingerichtet ist, dass sie den Stopper (108) drückt, um das Medizinprodukt beim Bewegen von der Aufbewahrungsposition in die Injektionsendposition auszustoßen,
eine Halterung (8), die in Bezug auf die Kolbenstange (4) zwischen einer Sperrposition, in der die Halterung (8) die Kolbenstange (4) in der Aufbewahrungsposition blockiert, und einer Freigabeposition, in der die Halterung (8) von der Kolbenstange (4) wegbewegt wird, um eine axiale Verschiebung der Kolbenstange (4) von der Aufbewahrungsposition in die Injektionsendposition zu ermöglichen, beweglich ist,
eine Verriegelung (9), die in Bezug auf die Halterung (8) zwischen einer Verriegelungsposition, in der die Verriegelung (9) die Halterung (8) in der Sperrposition verriegelt, und einer Freigabeposition, in der die Verriegelung (9) eine Bewegung der Halterung (8) in die Freigabeposition ermöglicht, wobei die Bewegung der Verriegelung (9) von der Verriegelungsposition in die Freigabeposition verursacht wird, indem sich die Nadelabdeckung (5) von der ersten ausgefahrenen Position in die zurückgezogene Position bewegt, beweglich ist,
eine in Bezug auf das Gehäuse (2) bewegliche Steuerung (7) zwischen einer Aufbewahrungsposition, in der die Steuerung (7) die Aktivierung des Autoinjektors (1) verhindert, und einer aktivierten Position, die die Aktivierung des Autoinjektors (1) ermöglicht, wobei die Steuerung (7) ein Greifelement (73) enthält, das so eingerichtet ist, dass es einem Benutzer ermöglicht, die Steuerung (7) von der Aufbewahrungsposition in die aktivierte Position zu bewegen,
wobei die Verriegelung (9) mit der Steuerung (7) verbunden ist, sodass die Bewegung der Steuerung (7) in Bezug auf das Gehäuse (2) zwischen der Aufbewahrungs- und einer aktivierten Position eine Bewegung der Verriegelung (9) in Bezug auf das Gehäuse (2) zwischen einer entsprechenden Aufbewahrungs- und einer aktivierten Position mit sich bringt, und
die Verriegelung (9) ein Sperrelement (96) enthält, das so eingerichtet ist, dass es an dem Anschlag (281) des Gehäuses (2) anliegt, solange sich die Verriegelung (9) in der Aufbewahrungsposition befindet, sodass das Bewegen der Verriegelung (9) in die Freigabeposition in Bezug auf die Halterung (8) verhindert ist, wobei das Sperrelement (96) der Verriegelung (9) von dem Anschlag (281) des Gehäuses (2) wegbewegt wird, wenn die Verriegelung (9) in die aktivierte Position bewegt wird, sodass die Verriegelung (9) sich in die Freigabeposition zur Halterung (8) bewegen kann.

2. Autoinjektor (1) nach dem vorhergehenden Anspruch, wobei die Steuerung (7) einen distalen Flansch (74) enthält, der eine distale Seite (740) und eine proximale Seite (741) aufweist, und das Gehäuse (2) eine proximale Lagerfläche (260, 270, 280) enthält, die zum axialen Anliegen an der distalen Seite (740) des distalen Flansches (74) angeordnet ist, und eine distale Lagerfläche (290) zum axialen Anliegen an der proximalen Seite (741) des distalen Flansches (74).

3. Autoinjektor (1) nach dem vorhergehenden Anspruch, wobei die proximale Lagerfläche (260) an einer axial erstreckenden Indexierrippe (26) angeordnet ist, wobei die Indexierrippe (26) axial gegenüber der proximalen Lagerfläche (260) ein distales Ende (261) enthält, das zum Eingreifen in einen axialen Schlitz (31) eines Bodenkörpers (3) des Gehäuses (2) eingerichtet ist.

4. Autoinjektor (1) nach dem vorhergehenden Anspruch 2, wobei die proximale Lagerfläche (280) an einer axial erstreckenden Sperrrippe (28) angeordnet ist, wobei die Sperrrippe (28) axial gegenüber der proximalen Lagerfläche (280) den distalen Anschlag (281) enthält, der so eingerichtet ist, dass er axial an der Verriegelung (9) anliegt.

5. Autoinjektor (1) nach dem vorhergehenden Anspruch 2, wobei die proximale Lagerfläche (270) an einer axial erstreckenden Montagerippe (27) angeordnet ist, wobei die Montagerippe (27) axial gegenüber der proximalen Lagerfläche (270) ein distales Ende (271) enthält, das so eingerichtet ist, dass es axial an einem proximalen Ende (30) eines Bodenkörpers (3) des Gehäuses (2) anliegt.

6. Autoinjektor (1) nach einem der vorhergehenden Ansprüche, wobei die Steuerung (7) einen proximalen Flansch (75) enthält, der zum Schließen eines geöffneten proximalen Endes (21) des Gehäuses (2) angeordnet ist, und das Gehäuse (2) eine proximale Lagerfläche (230) enthält, die so eingerichtet ist, dass sie axial an einer distalen Seite (751) des proximalen Flansches (75) anliegt.

7. Autoinjektor (1) nach den vorhergehenden Ansprüchen 2 und 6, wobei die proximale Lagerfläche (230), die so eingerichtet ist, dass sie axial an einer distalen Seite (751) des proximalen Flansches (75) anliegt, an einer ersten axial erstreckenden Rippe (23) des Gehäuses (2) angeordnet ist, und eine Peripheriekante (742) des distalen Flansches (74) der Steuerung (7) eine Aussparung (743) zur Aufnahme der ersten axial erstreckenden Rippe (23) durch den distalen Flansch (74) enthält.

8. Autoinjektor (1) nach dem vorhergehenden Anspruch, wobei die Steuerung (7) ein Rotationsbegrenzerelement (76) enthält, das eine erste seitliche Seite (760), die so eingerichtet ist, dass sie orthoradial an der ersten axial erstreckenden Rippe (23) des Gehäuses (2) anliegt, wenn sich die Steuerung (7) in der Aufbewahrungsposition befindet, und eine zweite seitliche Seite (761) aufweist, die so eingerichtet ist, dass sie orthoradial an einem Vorsprung (29) des Gehäuses (2) anliegt, wenn sich die Steuerung (7) in der aktivierten Position befindet, und das Rotationsbegrenzerelement (76) den distalen Flansch (74) mit dem proximalen Flansch (75) der Steuerung (7) verbindet.

9. Autoinjektor (1) nach einem der vorhergehenden Ansprüche, wobei das Greifelement (73) der Steuerung (7) ein konisches Außenende (731) enthält, das so eingerichtet ist, dass es auf einen ersten oder zweiten Indikator (24, 25) des Gehäuses (2) zeigt, um dem Endbenutzer anzuzeigen, wenn sich die Steuerung (7) in der Aufbewahrungsposition oder aktivierten Position befindet.

10. Autoinjektor (1) nach einem der vorhergehenden Ansprüche, wobei die Steuerung (7) einen elastisch verformbaren Halter (763) enthält, der so eingerichtet ist, dass er an dem Gehäuse (2) anliegt, um die Steuerung (7) vor der Verwendung des Autoinjektors (1) in der Aufbewahrungsposition zu halten.

11. Autoinjektor (1) nach dem vorhergehenden Anspruch, wobei der elastisch verformbare Halter (763) so eingerichtet ist, dass er an einer ersten Seite einer ersten axial erstreckenden Rippe (23) des Gehäuses (2) anliegt und gegenüber der ersten Seite die erste axial erstreckende Rippe (23) des Gehäuses (2) eine geneigte Fläche (231), wie eine Fase, enthält, um es dem elastisch verformbaren Halter (763) zu ermöglichen, sich so zu verformen, dass er bei dem Aufbau der Steuerung (7) über die erste axial erstreckende Rippe (23) innerhalb des Gehäuses (2) hinweggeht.

12. Autoinjektor (1) nach einem der vorhergehenden Ansprüche, wobei die Halterung (8) einen axial erstreckenden Führungsschenkel (81) enthält, der so eingerichtet ist, dass er in eine axial erstreckende Führungsnut (95) der Verriegelung (9) eingreift, wobei der axiale Führungsschenkel (81) eine orthoradiale Schiebefläche (813) definiert, die so eingerichtet ist, dass sie an einer seitlichen Seite der Führungsnut (95) anliegt, um die Verriegelung (9) in die aktivierte Position zu drehen, wenn die Halterung (8) in die aktivierte Position gedreht wird.

13. Autoinjektor (1) nach dem vorhergehenden Anspruch, wobei die Halterung (8) einen elastisch verformbaren Inflektionspunkt (820) enthält, der am Führungsschenkel (81) zum axialen Anliegen an einem proximalen Ende (90) der Verriegelung (9) angeordnet ist, um dem Endnutzer eine taktile Anzeige zu geben, dass die Injektion auszulösen ist.

14. Autoinjektor (1) nach einem der vorhergehenden Ansprüche, wobei die Halterung (8) elastisch verformbare Sperrschenkel (83) enthält, die einen radial nach innen gerichteten Vorsprung (832), der so eingerichtet ist, dass er in eine distale Anlagfläche (44) der Kolbenstange (4) eingreift, wenn sich die Halterung (8) in der Sperrposition befindet, einen gegenüberliegenden radial nach außen gerichteten Vorsprung (834), der so eingerichtet ist, dass er in eine axial erstreckende Verriegelungsnut (94) der Verriegelung (9) eingreift, sodass der nach außen gerichtete Vorsprung (834) an einer seitlichen Anlagfläche (941) der Verriegelungsnut (94) anliegt, wenn sich die Verriegelung (9) in der Verriegelungsposition befindet, enthält, und wobei der nach außen gerichtete Vorsprung (834) ferner eine proximale Schulter (835) definiert, die so eingerichtet ist, dass sie axial an einem distalen Anschlag (943) anliegt, der radial nach innen aus der Verriegelungsnut (94) herausragt.

15. Autoinjektor (1) nach einem der vorhergehenden Ansprüche, wobei die Halterung (8) und die Steuerung (7) aus einem Stück bestehen.

## Revendications

1. Auto-injecteur (1), pour l'injection automatique d'un produit dans un site d'injection, ledit auto-injecteur (1) comprenant :
un boîtier (2) s'étendant le long d'un axe longitudinal A et configuré pour recevoir un récipient médical (100) ayant un cylindre (101) définissant un réservoir pour contenir un produit médical, ledit cylindre (101) ayant une extrémité distale (102) munie d'une aiguille d'injection et une extrémité proximale ouverte (103) configurée pour recevoir une tige de piston (4) pour pousser un bouchon (108) agencé à l'intérieur du cylindre (101), le boîtier (2) comprenant une butée (281),
un couvercle d'aiguille (5) couplé audit boîtier (2) et mobile de manière axiale par rapport à celui-ci entre une première position étendue, dans laquelle le couvercle d'aiguille (5) protège au moins partiellement l'aiguille d'injection, une position rétractée, dans laquelle le couvercle d'aiguille (5) se déplace de manière proximale par rapport au boîtier (2), et une seconde position étendue dans laquelle le couvercle d'aiguille (5) se déplace en arrière dans la direction distale pour protéger l'aiguille d'injection,
une tige de piston (4) mobile de manière axiale à l'intérieur du boîtier (2) entre une position de stockage et une position de fin d'injection située de manière distale par rapport à la position de stockage, la tige de piston (4) étant configurée pour pousser le bouchon (108) afin d'expulser le produit médical lors du déplacement de la position de stockage à la position de fin d'injection,
un dispositif de retenue (8), mobile par rapport à la tige de piston (4) entre une position de blocage, dans laquelle le dispositif de retenue (8) bloque la tige de piston (4) dans la position de stockage, et une position de libération, dans laquelle le dispositif de retenue (8) se déplace loin de la tige de piston (4) pour permettre le déplacement axial de la tige de piston (4) de la position de stockage à la position de fin d'injection,
un verrou (9), mobile par rapport au dispositif de retenue (8) entre une position de verrouillage, dans laquelle le verrou (9) verrouille le dispositif de retenue (8) dans la position de blocage, et une position de libération, dans laquelle le verrou (9) permet le mouvement du dispositif de retenue (8) vers la position de libération, le mouvement du verrou (9) de la position de verrouillage à la position de libération étant provoqué par le mouvement du couvercle d'aiguille (5) de la première position étendue à la position rétractée,
un dispositif de commande (7), mobile par rapport au boîtier (2), entre une position de stockage dans laquelle le dispositif de commande (7) empêche l'activation de l'auto-injecteur (1) et une position activée permettant l'activation de l'auto-injecteur (1), ledit dispositif de commande (7) comprenant un élément de préhension (73) configuré pour permettre à un utilisateur de déplacer le dispositif de commande (7) de la position de stockage à la position activée,
dans lequel le verrou (9) est relié au dispositif de commande (7) de sorte que le mouvement du dispositif de commande (7) par rapport au boîtier (2) entre la position de stockage et la position activée entraîne le mouvement du verrou (9) par rapport au boîtier (2) entre une position de stockage et une position activée correspondantes, et
le verrou (9) comprenant un élément de blocage (96) configuré pour venir en butée contre la butée (281) du boîtier (2) tant que le verrou (9) se trouve dans la position de stockage, de sorte que le verrou (9) soit empêché de se déplacer vers la position de libération par rapport au dispositif de retenue (8), l'élément de blocage (96) du verrou (9) se déplaçant loin de ladite butée (281) du boîtier (2) lorsque le verrou (9) se déplace vers la position activée, de sorte que le verrou (9) puisse se déplacer vers la position de libération par rapport au dispositif de retenue (8).

2. Auto-injecteur (1) selon la revendication précédente, dans lequel le dispositif de commande (7) comprend une bride distale (74) ayant un côté distal (740) et un côté proximal (741), et le boîtier (2) comprend une surface d'appui proximale (260, 270, 280) agencée pour venir en butée de manière axiale contre le côté distal (740) de ladite bride distale (74), et une surface d'appui distale (290) pour venir en butée de manière axiale contre le côté proximal (741) de la bride distale (74).

3. Auto-injecteur (1) selon la revendication précédente, dans lequel la surface d'appui proximale (260) est agencée au niveau d'une nervure d'indexation (26) s'étendant de manière axiale, ladite nervure d'indexation (26) comprenant, axialement à l'opposé de la surface d'appui proximale (260), une extrémité distale (261) configurée pour s'engager avec une fente axiale (31) d'un corps inférieur (3) du boîtier (2).

4. Auto-injecteur (1) selon la revendication précédente 2, dans lequel la surface d'appui proximale (280) est agencée au niveau d'une nervure de blocage (28) s'étendant de manière axiale, ladite nervure de blocage (28) comprenant, axialement à l'opposé de la surface d'appui proximale (280), la butée distale (281) configurée pour venir en butée de manière axiale contre le verrou (9).

5. Auto-injecteur (1) selon la revendication précédente 2, dans lequel la surface d'appui proximale (270) est agencée au niveau d'une nervure de montage (27) s'étendant de manière axiale, ladite nervure de montage (27) comprenant, axialement à l'opposé de la surface d'appui proximale (270), une extrémité distale (271) configurée pour venir en butée de manière axiale contre une extrémité proximale (30) d'un corps inférieur (3) du boîtier (2).

6. Auto-injecteur (1) selon l'une des revendications précédentes, dans lequel le dispositif de commande (7) comprend une bride proximale (75) agencée pour fermer une extrémité proximale ouverte (21) du boîtier (2), et le boîtier (2) comprend une surface d'appui proximale (230) configurée pour venir en butée de manière axiale contre un côté distal (751) de ladite bride proximale (75).

7. Auto-injecteur (1) selon les revendications précédentes 2 et 6, dans lequel la surface d'appui proximale (230) configurée pour venir en butée de manière axiale contre un côté distal (751) de ladite bride proximale (75) est agencée au niveau d'une première nervure (23) s'étendant de manière axiale du boîtier (2), et un bord périphérique (742) de la bride distale (74) du dispositif de commande (7) comprend une découpe (743) pour recevoir la première nervure (23) s'étendant de manière axiale à travers la bride distale (74).

8. Auto-injecteur (1) selon la revendication précédente, dans lequel le dispositif de commande (7) comprend un élément de limitation de rotation (76) ayant un premier côté latéral (760) configuré pour venir en butée de manière orthoradiale contre la première nervure (23) s'étendant de manière axiale du boîtier (2) lorsque le dispositif de commande (7) est dans la position de stockage, et un second côté latéral (761) configuré pour venir en butée de manière orthoradiale contre une saillie (29) du boîtier (2) lorsque le dispositif de commande (7) est dans la position activée, et l'élément de limitation de rotation (76) relie la bride distale (74) à la bride proximale (75) du dispositif de commande (7).

9. Auto-injecteur (1) selon l'une des revendications précédentes, dans lequel l'élément de préhension (73) du dispositif de commande (7) comprend une extrémité externe effilée (731) configurée pour pointer vers un premier ou un second indicateur (24, 25) du boîtier (2) afin d'indiquer à l'utilisateur final le moment où le dispositif de commande (7) se trouve dans la position de stockage ou la position activée.

10. Auto-injecteur (1) selon l'une des revendications précédentes, dans lequel le dispositif de commande (7) comprend un support déformable élastiquement (763) configuré pour venir en butée contre le boîtier (2) afin de maintenir le dispositif de commande (7) dans la position de stockage avant l'utilisation de l'auto-injecteur (1).

11. Auto-injecteur (1) selon la revendication précédente, dans lequel le support déformable élastiquement (763) est configuré pour venir en butée contre un premier côté d'une première nervure (23) s'étendant de manière axiale du boîtier (2) et, à l'opposé dudit premier côté, la première nervure (23) s'étendant de manière axiale du boîtier (2) comprend une surface inclinée (231), telle qu'un chanfrein, pour permettre au support déformable élastiquement (763) de se déformer de manière à passer sur la première nervure (23) s'étendant de manière axiale lors de l'assemblage du dispositif de commande (7) à l'intérieur du boîtier (2).

12. Auto-injecteur (1) selon l'une des revendications précédentes, dans lequel le dispositif de retenue (8) comprend une patte de guidage (81) s'étendant de manière axiale configurée pour s'engager avec une rainure de guidage (95) s'étendant de manière axiale du verrou (9), la patte de guidage (81) s'étendant de manière axiale définissant une surface de poussée orthoradiale (813) configurée pour venir en butée contre un côté latéral de la rainure de guidage (95) afin de faire tourner le verrou (9) vers la position activée lorsque le dispositif de retenue (8) tourne vers la position activée.

13. Auto-injecteur (1) selon la revendication précédente, dans lequel le dispositif de retenue (8) comprend un point d'inflexion (820) déformable élastiquement agencé au niveau de la patte de guidage (81) pour venir en butée de manière axiale contre une extrémité proximale (90) du verrou (9) afin de fournir à l'utilisateur final une indication tactile selon laquelle l'injection est sur le point d'être déclenchée.

14. Auto-injecteur (1) selon l'une des revendications précédentes, dans lequel le dispositif de retenue (8) comprend des pattes de blocage (83) déformables élastiquement comprenant une saillie radialement vers l'intérieur (832) configurée pour s'engager avec une surface de butée distale (44) de la tige de piston (4) lorsque le dispositif de retenue (8) se trouve dans la position de blocage, une saillie opposée radialement vers l'extérieur (834) configurée pour s'engager avec une rainure de verrouillage (94) s'étendant de manière axiale du verrou (9) de sorte que la saillie vers l'extérieur (834) vienne en butée contre une surface de butée latérale (941) de ladite rainure de verrouillage (94) lorsque le verrou (9) est dans la position de verrouillage, et dans lequel la saillie vers l'extérieur (834) définit en outre un épaulement proximal (835) configuré pour venir en butée de manière axiale contre une butée distale (943) faisant saillie radialement vers l'intérieur depuis la rainure de verrouillage (94).

15. Auto-injecteur (1) selon l'une des revendications précédentes, dans lequel le dispositif de retenue (8) et le dispositif de commande (7) sont réalisés d'une seule pièce.
